# EUROPÄISCHE PATENTSCHRIFT

(11) **EP 1 286 950 B1**
(45) Veröffentlichungstag und Bekanntmachung des Hinweises auf die Patenterteilung: **16.11.2005**
(21) Anmeldenummer: 01951474.4
(22) Anmeldetag: 03.05.2001
(51) Int. Cl.: C07C 213/00, C07C 217/64

(54) **VERFAHREN ZUR HERSTELLUNG VON ARYLPOLY(OXALKYL)-BENZYLDIMETHYL-AMMONIUMDERIVATEN**
METHOD FOR PRODUCING ARYLPOLY(OXALKYL)-BENZYLDIMETHYL-AMMONIUM DERIVATIVES
PROCEDE DE FABRICATION DE DERIVES D'ARYLPOLY(OXALKYLE)-BENZYLDIMETHYLAMMONIUM

(30) Priorität: 16.05.2000 DE 10023955
(43) Veröffentlichungstag der Anmeldung: 05.03.2003
(73) Patentinhaber: Clariant GmbH, 65929 Frankfurt am Main (DE)
(72) Erfinder: RAAB, Klaus, 84508 Burgkirchen (DE); CRASS, Gerhard, 61169 Friedberg (DE); AIGNER, Rudolf, 84556 Kastl (DE)
(74) Vertreter: Mikulecky, Klaus
(86) Internationale Anmeldenummer: PCT/EP2001/004963
(87) Internationale Veröffentlichungsnummer: WO 2001/087818

(56) Entgegenhaltungen:
- US-A- 2 115 250

## Beschreibung

Die vorliegende Erfindung betrifft ein neues Verfahren zur Herstellung von Arylpoly(oxalkyl)benzyldimethylammoniumderivaten durch Umsetzung von Arylpoly(oxalkyl)verbindungen mit Benzyldimethylamin oder substituierten Benzyldimethylaminen in einem geeigneten Solvens.

Einzelne Vertreter aus der Verbindungsklasse der Arylpoly(oxalkyl)benzyldimethylammoniumhalogenide wie beispielsweise Benzethoniumchlorid 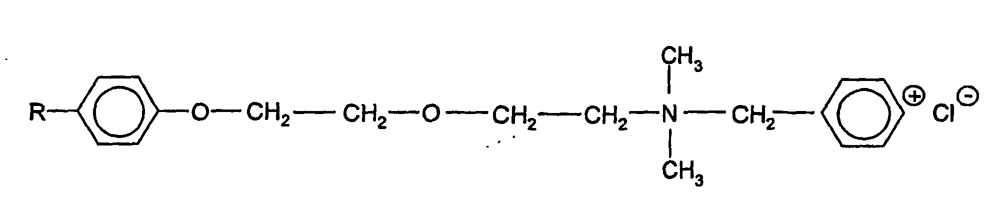 oder Methylbenzethoniumchlorid 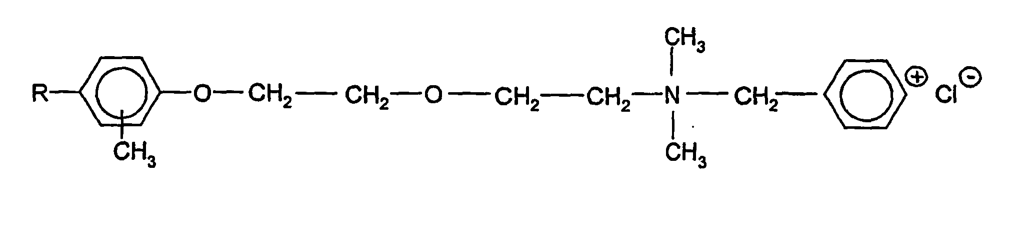 mit

R = (CH₃)₃C-CH₂-C(CH₃)₂-

sind seit langem als Biozide kommerziell im Einsatz. Bei ihnen handelt es sich um kristalline Pulver, deren Reinheitsanforderungen in verschiedenen Pharmakopoen beschrieben sind. Auch Lösungen dieser kristallinen Pulver werden eingesetzt.

Diese Verbindungen haben bakterizide und fungizide Wirkung. Ihre Anwendungsgebiete als wirksamer Bestandteil von Formulierungen sind zum Beispiel die Desinfektion im Krankenhausbereich, im pharmazeutischen Bereich oder im Veterinär- und Nahrungsmittelsektor. Außerdem werden sie verwendet als Konservierungsmittel für Kosmetika wie Haarconditioner, Reinigungslotionen oder Shampoos, vor allem in rinse-off Produkten sowie als Mittel zur Geruchsverhinderung.

Gemäß Workbook for Organic Synthesis: The Disconnection Approach, Stuart Warren, John Wiley & Sons, 1982, auf Seite 49 - 51 verfährt man zur Herstellung solcher Verbindungen mit Y = H und R' = H nach Stand der Technik wie in den Reaktionsgleichungen 1 bis 3 angegeben. 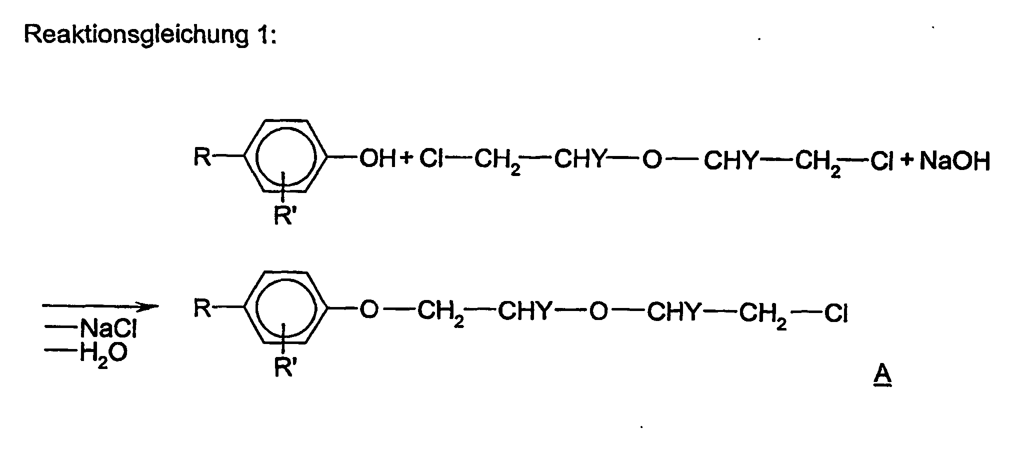 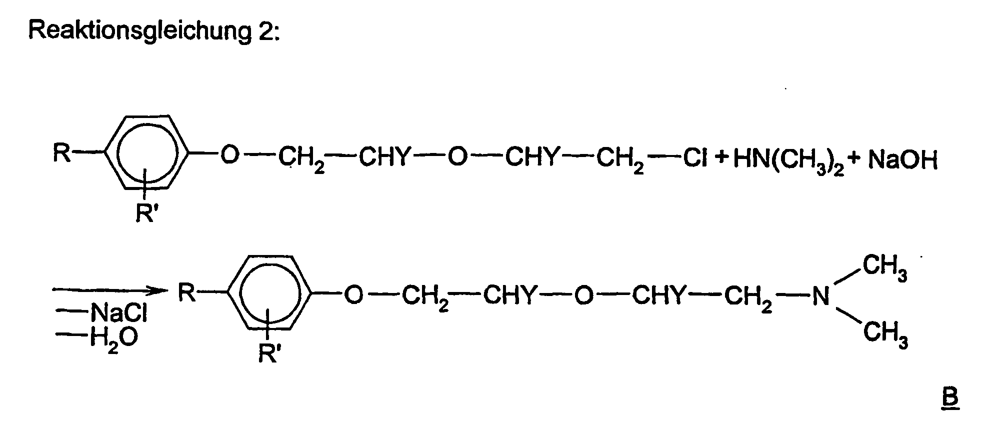 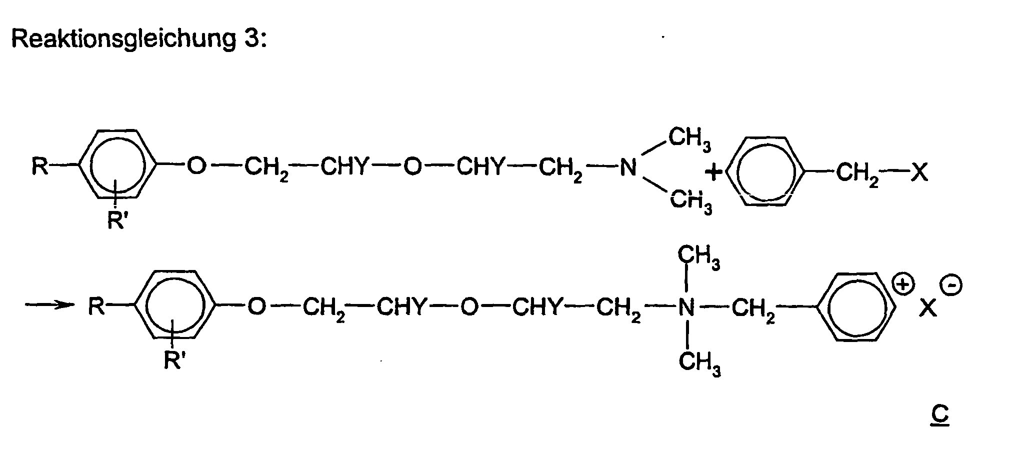 worin
R Alkyl
R' H, Alkyl
Y H, CH₃ oder Alkyl
X Cl, Br bedeuten

US-2 098 203 offenbart Arylpoly(oxalkyl)chloride der Formel A mit Y = H oder C₁- bis C₅-Alkyl, R = C₈- bis C₁₈-Alkyl und R' = H, Alkyl oder Alkoxy. Verbindungen dieses Typs stellen die Ausgangsstoffe des erfindungsgemäßen Verfahrens dar.

US-2 170 111 offenbart tertiäre Amine der Formel B und weitere tertiäre Amine von ähnlicher Struktur wie B. Deren Herstellung erfolgt analog zu Reaktionsgleichung 2 durch Umsetzung der Arylpoly(oxalkyl)chloride der Formel A mit Aminen wie beispielsweise Dimethylamin, Diethylamin, Diethanolamin oder Morpholin, basische Aufarbeitung, Wäsche und nachfolgende Vakuumdestillation.

Arylpoly(oxalkyl)benzyldimethylammoniumchloride der Formel C sowie ähnlich substituierte quaternäre Ammoniumkationen mit weiteren Anionen werden in US-2 115 250 offenbart. Sie werden durch Umsetzung der tertiären Amine der Formel B oder tertiärer Amine mit ähnlicher Struktur wie B mit Alkylierungsmitteln wie beispielsweise Benzylchlorid, Dimethylsulfat, Diethylsulfat, Dimethyloxalat, Methyliodid oder Ethylbromid analog zu Reaktionsgleichung 3 erhalten.

Im Journal of the American College of Toxicology, Volume 4, Number 5, 1985 auf Seite 66 wird als Herstellweg für Benzethoniumchlorid und Methylbenzethoniumchlorid ebenfalls die Umsetzung der entsprechenden tertiären Amine mit Benzylchlorid bei 60 - 80°C angegeben.

Wegen der Aufwendigkeit dieser mehrstufigen Reaktionssequenz einschließlich der erforderlichen Aufarbeitungsschritte, und wegen des unerwünschten stöchiometrischen Anfalls von Natriumchlorid oder ähnlicher Salze bestand nun die vorliegender Erfindung zugrunde liegende Aufgabe darin, ein vereinfachtes Herstellverfahren für Arylpoly(oxalkyl)benzyldimethylammoniumderivate, insbesondere für Benzethoniumchlorid oder Methylbenzethoniumchlorid, zu entwickeln.

Überraschenderweise wurde gefunden, daß mit einem Verfahren, bei dem Arylpoly(oxalkyl)verbindungen der Formel (2) mit Benzyldimethylamin oder substituierten Benzyldimethylaminen der Formel (3) in einem geeigneten Solvens zu den Arylpoly(oxalkyl)benzyldimethylammoniumderivaten der Formel (1) umgesetzt werden, diese Aufgabe gelöst werden kann.

Gegenstand der Erfindung ist daher ein Verfahren zur Herstellung von Arylpoly(oxalkyl)benzyldimethylammoniumderivaten der Formel (1) 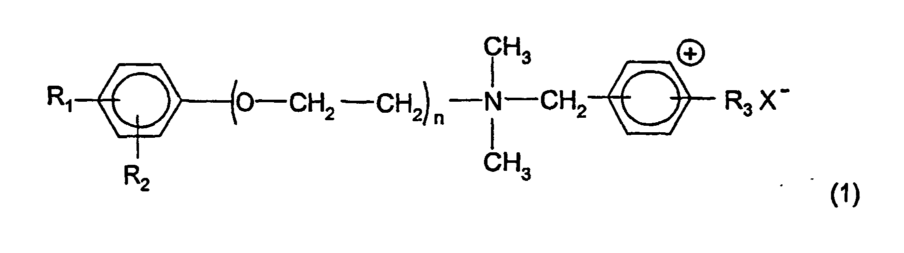 durch Umsetzung von Verbindungen der Formeln (2) und (3) 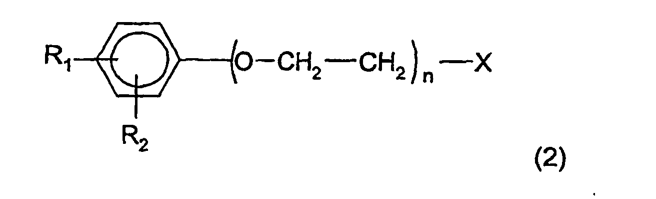 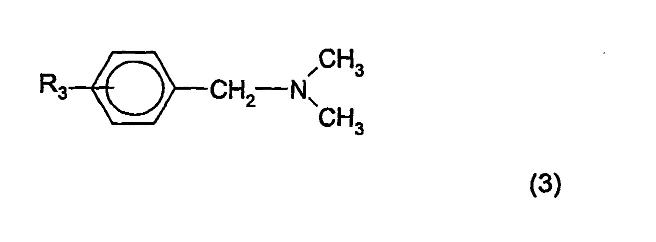 worin
X = Br, Cl, R-SO₃ mit R = Alkyl, Alkylaryl
R₁ = H, C₁- bis C₁₆-Alkyl oder O-C₁- bis O-C₁₆-Alkyl in ortho-, meta- oder para-Position,
R₂= H, C₁- bis C₄-Alkyl oder O-C₁- bis O-C₄-Alkyl,
R₃= H, C₁- bis C₁₆-Alkyl oder O-C₁- bis O-C₁₆-Alkyl in ortho-, meta- oder para-Position, und
n = 1, 2, 3 oder 4 bedeuten,
bei Temperaturen von 60 bis 160°C in einem Lösungsmittel unter dem sich dabei im geschlossenen Reaktionsgefäß einstellenden Druck.

R₁ steht vorzugsweise für C₄- bis C₁₂-Alkyl, insbesondere für Octyl oder Isooctyl in para-Position. R₂ steht vorzugsweise für H oder CH₃. n steht vorzugsweise für 2. R₃ steht vorzugsweise für H. X steht vorzugsweise für Cl.

Für R₁ = (CH₃)₃C-CH₂-C(CH₃)₂ in para-Stellung, R₂ = H, n = 2, R₃ = H, X = Cl wird Benzethoniumchlorid und für R₁ = (CH₃)₃C-CH₂-C(CH₃)₂ in para-Stellung, R₂ = CH₃, n = 2, R₃ = H, X = Cl wird Methylbenzethoniumchlorid erhalten. Die Verfahren zur Herstellung dieser Verbindungen stellen bevorzugte Ausführungsformen der Erfindung dar.

Für das erfindungsgemäße Verfahren geeignete Solventien sind vorzugsweise polare Solventien, wie beispielsweise kurzkettige Alkohole mit 1 bis 6, vorzugsweise 1 bis 4 C-Atomen, die ein- oder mehrwertig sein können, beispielsweise Methanol, Ethanol, Isopropanol oder Monoethylenglykol, deren Mischungen mit Wasser und insbesondere Wasser selbst.

Vorzugsweise werden die Arylpoly(oxalkyl)verbindungen der Formel (2) und das Benzyldimethylamin oder die substituierten Benzyldimethylamine der Formel (3) im Molverhältnis (2) : (3) = 1 : 0,95 bis 1 : 1,05 eingesetzt. Die Umsetzung wird bei Temperaturen im Bereich von vorzugsweise 100 bis 140°C, insbesondere bei 115 bis 130°C, unter dem sich einstellenden Druck der Reaktionsmischung durchgeführt. Die Reaktionszeiten werden vorzugsweise so gewählt, daß die nach Probenahmen mittels HPLC bestimmten Konzentrationen der Ausgangsverbindungen der Formel (2) und (3) möglichst niedrig sind.

Das aus der Reaktion erhaltene Arylpoly(oxalkyl)benzyldimethylammoniumderivat der Formel (1) kann durch Umkristallisation, beispielsweise aus Chloroform, Ether, Toluol oder Xylol gereinigt werden. Vorzugsweise wird vor der Umkristallisation das Solvens abgetrennt.

Falls Wasser als das besonders bevorzugte Solvens bei der Umsetzung eingesetzt worden ist, kann zur Aufarbeitung nach Reaktionsende das Wasser beispielsweise durch Azeotropdestillation, Vakuumtrocknung, Sprühtrocknung oder Wirbelschichttrocknung abgetrennt werden. Das rohe, wasserfreie Arylpoly(oxalkyl)benzyldimethylammoniumderivat der Formel (1) kann dann durch Umkristallisation, beispielsweise aus Chloroform, Ether, Toluol oder Xylol gereinigt werden.

Die Erfindung wird nun an Beispielen noch näher erläutert.

### Beispiel 1

### 1396 g 2-(2-chlorethoxy)ethyl-p-isooctylphenylether der Formel

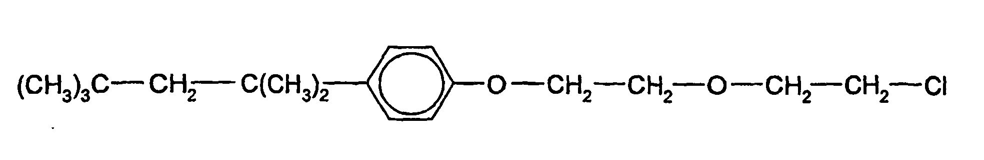

603 g Benzyldimethylamin und 1000 g vollentsalztes Wasser werden in einem 5 Liter-Autoklaven unter einer Stickstoffatmosphäre 20 Stunden bei 120°C und einem Druck von 1,5 - 2,0 bar gerührt. Die anfangs zweiphasige Reaktionsmischung wird im Laufe der Umsetzung einphasig. Die Reaktionsmischung wird nach dem Entspannen und Abkühlen auf etwa 80°C in einen 10 Liter-Glasrührkolben überführt und mit 3000 g Toluol versetzt. Unter Rühren wird das Wasser zusammen mit Toluol azeotrop abdestilliert. Das farblose Destillat trennt sich auf in eine toluolhaltige Oberphase, die in den 10 Liter-Glasrührkolben zurückgeführt wird und in eine wäßrige Unterphase, die abgetrennt wird. Die gelbe, klare wasserfreie Toluol-Lösung wird bei etwa 110°C heiß über ein beheiztes Druckfilter filtriert. Das heiße Filtrat wird unter einer Stickstoffatmosphäre unter langsamem Rühren und Impfkristallzugabe langsam auf 30°C abgekühlt. Der entstandene Kristallbrei wird über eine Nutsche abgesaugt und die abgenutschten Kristalle mehrmals mit Toluol gewaschen. Die abgenutschten, feinen weißen Kristalle werden bei 80°C im Vakuum getrocknet. Die Ausbeute nach Trocknung beträgt 70 %. Das Produkt hat einen Schmelzpunkt von 162°C und der durch alkalische Zweiphasentitration ermittelte Gehalt liegt über 99 %.

### Beispiel 2

### 443 g 2-(2-chlorethoxy)ethyl-p-isooctylphenylether der Formel

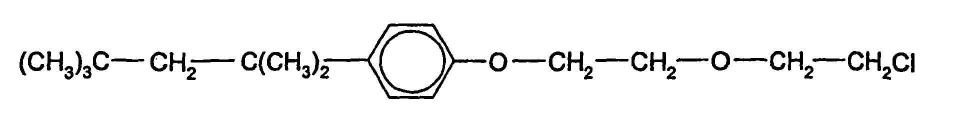

192 g Benzyldimethylamin und 320 g destilliertes Wasser werden in einem Autoklaven unter einer Stickstoffatmosphäre 15 Stunden bei 120 -125°C und einem Druck von 1,5 - 2,0 bar gerührt. Die anfangs zweiphasige Reaktionsmischung wird im Laufe der Umsetzung einphasig. Der Autoklav wird entspannt und die warme Reaktionsmischung unter Dosierung in einem Trockner bei etwa 80°C im Vakuum vom Wasser befreit. Das nun feste, wasserfreie Rohprodukt wird aus 950 g Toluol umkristallisiert, mit Toluol mehrmals gewaschen und bei 80°C im Vakuum getrocknet. Die Ausbeute an feinen weißen Kristallen beträgt 72 % und der mittels HPLC bestimmte Gehalt liegt bei ≥ 98 %,

### Beispiele 3 bis 11

87,3 g 2-(2-chlorethoxy)ethyl-p-isooctylphenylether (a), 37,7 g Benzyldimethylamin und Solvens (Menge und Art siehe Tabelle) werden in einem Autoklaven unter einer Stickstoffatmosphäre bei dem sich einstellenden Eigendruck gerührt. Die Reaktionsbedingungen sind in der Tabelle aufgeführt. Vom jeweiligen Rohprodukt werden Proben gezogen und mittels HPLC analysiert.

| Beispiel | Menge Solvens | Reaktionstemperatur [°C] | Reaktionsdauer [h] | Ausbeute an nicht isoliertem Produkt laut HPLC [%] | Umsatz an (a) laut HPLC [%] |
|---|---|---|---|---|---|
| 3 | 125 g Wasser | 135 | 4 | 70 | 89 |
| 4 | 125 g Wasser | 135 | 7 | 72 | 95 |
| 5 | 125 g Wasser | 115 | 7 | 56 | 63 |
| 6 | 125 g Wasser | 115 | 23 | 80 | 95 |
| 7 | 250 g Wasser | 120 | 7 | 66 | 75 |
| 8 | 250 g Wasser | 120 | 20 | 75 | 97 |
| 9 | 375 g Wasser | 120 | 20 | 76 | 97 |
| 10 | 125 g Methanol | 120 | 8 | 52 | 72 |
| 11 | 125 g Monoethylenglykol | 120 | 8 | 65 | 86 |

Die Aufarbeitung und Reinigung erfolgt analog zu Beispiel 2 durch Abziehen des jeweiligen Solvens im Vakuum, nachfolgende Umkristallisation aus Toluol oder Xylol und Vakuumtrocknung der abgenutschten weißen Kristalle.

## Patentansprüche

1. Verfahren zur Herstellung von Arylpoly(oxalkyl)benzyldimethylammoniumderivaten der Formel (1) 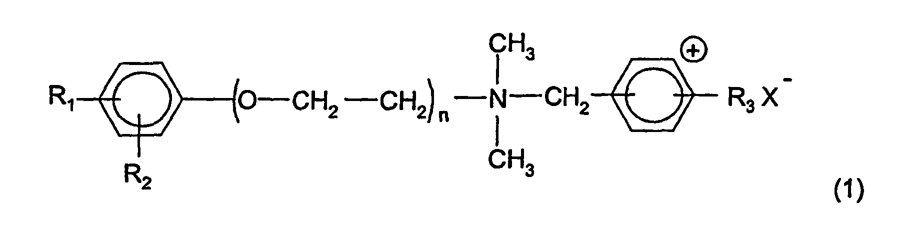 durch Umsetzung von Verbindungen der Formeln (2) und (3) 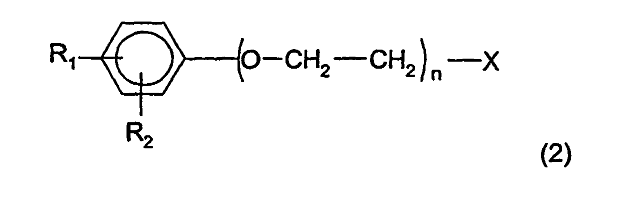 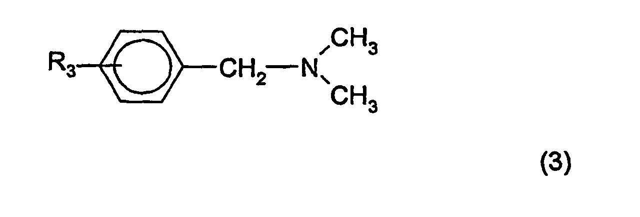 worin
X = Br, Cl, R-SO₃ mit R = Alkyl, Alkylaryl
R₁ = H, C₁- bis C₁₆-Alkyl oder O-C₁- bis O-C₁₆-Alkyl in ortho-, meta- oder para-Position,
R₂= H, C₁- bis C₄-Alkyl oder O-C₁- bis O-C₄-Alkyl,
R₃= H, C₁- bis C₁₆-Alkyl oder O-C₁- bis O-C₁₆-Alkyl in ortho-, meta- oder para-Position, und
n = 1, 2, 3 oder 4 bedeuten,
bei Temperaturen von 60 bis 160°C in einem Lösungsmittel unter dem sich dabei im geschlossenen Reaktionsgefäß einstellenden Druck.

2. Verfahren gemäß Anspruch 1, bei dem die Umsetzung von (2) und (3) in einem polaren Solvens durchgeführt wird.

3. Verfahren gemäß Anspruch 1 und/oder 2, bei dem das Solvens ausgewählt ist aus der Gruppe bestehend aus kurzkettigen Alkoholen mit 1 bis 6 C-Atomen, Wasser oder deren Mischungen.

4. Verfahren gemäß einem oder mehreren der Ansprüche 1 bis 3, bei dem R₁ für C₄- bis C₁₂-Alkyl steht.

5. Verfahren gemäß einem oder mehreren der Ansprüche 1 bis 4, bei dem R₁ für Octyl oder Isooctyl in para-Position steht.

6. Verfahren gemäß einem oder mehreren der Ansprüche 1 bis 5, bei dem R₂ für H oder CH₃ steht.

7. Verfahren gemäß einem oder mehreren der Ansprüche 1 bis 6, bei dem n für 2 steht.

8. Verfahren gemäß einem oder mehreren der Ansprüche 1 bis 7, bei dem R₃ für H und X für Cl steht.

9. Verfahren gemäß einem oder mehreren der Ansprüche 1 bis 8, bei dem die Arylpoly(oxalkyl)verbindungen der Formel (2) und die Benzyldimethylamine der Formel (3) im Molverhältnis 1 : 0,95 bis 1 : 1,05 eingesetzt werden.

10. Verfahren gemäß einem oder mehreren der Ansprüche 1 bis 9, bei dem die Umsetzung von (2) und (3) bei Temperaturen von 100 bis 140°C, insbesondere von 115 bis 130°C, unter dem autogenen Druck der Reaktionsmischung durchgeführt wird.

11. Verfahren gemäß einem oder mehreren der Ansprüche 1 bis 10, **dadurch gekennzeichnet, daß** das bei der Umsetzung von (2) und (3) verwendete Solvens nach der Umsetzung abgetrennt und das Arylpoly(oxalkyl)benzyldimethylammoniumderivat der Formel (1) durch Umkristallisieren gereinigt wird.

12. Verfahren gemäß Anspruch 11, **dadurch gekennzeichnet, daß** im Falle der Verwendung von Wasser als Solvens bei der Umsetzung von (2) und (3) das Wasser nach der Umsetzung beispielsweise durch Azeotropdestillation mittels Toluol oder Xylol, Vakuumtrocknung, Sprühtrocknung oder Wirbelschichttrocknung abgetrennt wird und das Arylpoly(oxalkyl)benzyldimethylammoniumderivat der Formel (1) durch Umkristallisieren gereinigt wird.

## Claims

1. A process for preparing arylpoly(oxalkyl)benzyldimethylammonium derivatives of the formula (1) 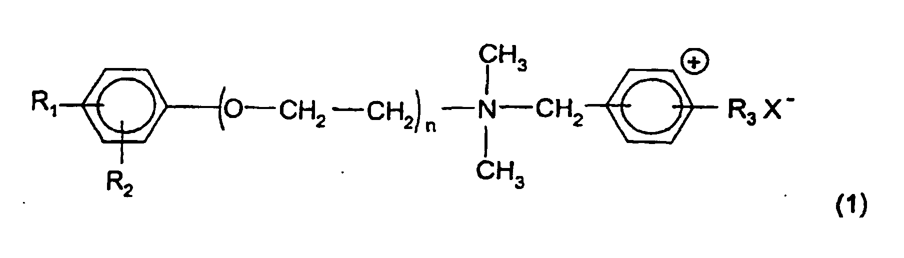 which comprises reacting compounds of the formulae (2) and (3) 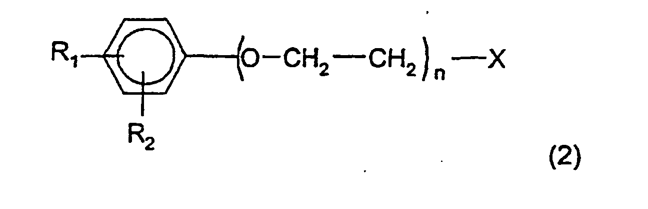 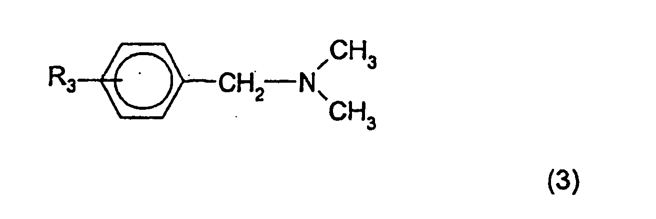 where
X = Br, Cl, R-SO₃ with R = alkyl, alkylaryl
R₁ = H, C₁- to C₁₆-alkyl or O-C₁- to O-C₁₆-alkyl in the ortho, meta or para position,
R₂ = H, C₁- to C₄-alkyl or O-C₁- to O-C₄-alkyl,
R₃ = H, C₁- to C₁₆-alkyl or O-C₁- to O-C₁₆-alkyl in the ortho, meta or para position, and
n = 1, 2, 3 or 4,
at temperatures of from 60 to 160°C in a solvent under the autogenous pressure in the closed reaction vessel.

2. The process as claimed in claim 1, wherein the reaction of (2) and (3) is carried out in a polar solvent.

3. The process as claimed in claim 1 and/or 2, wherein the solvent is selected from the group consisting of short-chain alcohols having from 1 to 6 carbon atoms, water and mixtures thereof.

4. The process as claimed in one or more of claims 1 to 3, wherein R₁ is C₄- to C₁₂-alkyl.

5. The process as claimed in one or more of claims 1 to 4, wherein R₁ is octyl or isooctyl in the para position.

6. The process as claimed in one or more of claims 1 to 5, wherein R₂ is H or CH₃.

7. The process as claimed in one or more of claims 1 to 6, wherein n is 2.

8. The process as claimed in one or more of claims 1 to 7, wherein R₃ is H and X is Cl.

9. The process as claimed in one or more of claims 1 to 8, wherein the arylpoly(oxalkyl) compounds of the formula (2) and the benzyldimethylamines of the formula (3) are used in a molar ratio of from 1:0.95 to 1:1.05.

10. The process as claimed in one or more of claims 1 to 9, wherein the reaction of (2) and (3) is carried out at temperatures of from 100 to 140°C, in particular from 115 to 130°C, under the autogenous pressure of the reaction mixture.

11. The process as claimed in one or more of claims 1 to 10, wherein the solvent used in the reaction of (2) and (3) is removed after the reaction and the arylpoly(oxalkyl)benzyldimethylammonium derivative of the formula (1) is purified by recrystallization.

12. The process as claimed in claim 11, wherein water is used as the solvent in the reaction of (2) and (3) and is removed after the reaction, for example, by azeotropic distillation using toluene or xylene, vacuum drying, spray drying or fluidized-bed drying, and the arylpoly(oxalkyl)benzyldimethylammonium derivative of the formula (1) is purified by recrystallization.

## Revendications

1. Procédé de préparation de dérivés de l'arylpoly(oxalkyl)benzyldiméthylammonium de formule (1) 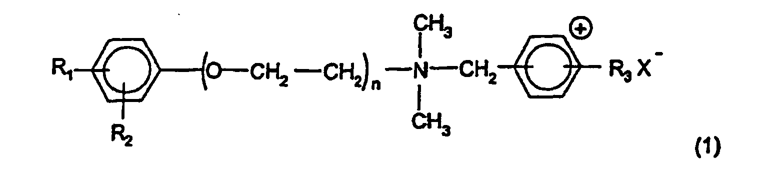 par réaction de composés ayant les formules (2) et (3) 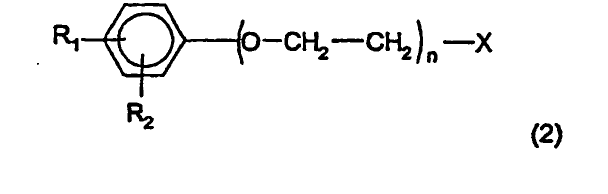 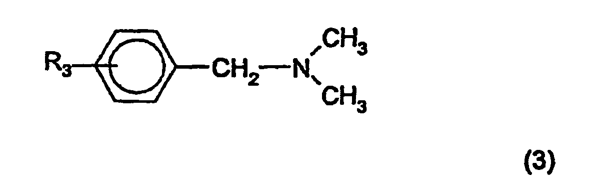 dans lesquelles
X est Br, Cl, R-SO₃, R étant un groupe alkyle ou alkylaryle,
R₁ est H ou un groupe alkyle en C₁ à C₁₆ ou O- (alkyle en C₁ à C₁₆) en position ortho, méta ou para,
R₂ est H ou un groupe alkyle en C₁ à C₄ ou O- (alkyle en C₁ à C₄),
R₃ est H ou un groupe alkyle en C₁ à C₁₆ ou O- (alkyle en C₁ à C₁₆) en position ortho, méta ou para, et
n vaut 1, 2, 3 ou 4,
à des températures de 60 à 160°C dans un solvant sous la pression qui se crée alors dans le réacteur fermé.

2. Procédé selon la revendication 1, dans lequel la réaction de (2) et de (3) est mise en oeuvre dans un solvant polaire.

3. Procédé selon les revendications 1 et/ou 2, dans lequel le solvant est choisi dans le groupe consistant en les alcools à courte chaîne ayant de 1 à 6 atomes de carbone, l'eau ou les mélanges de ceux-ci.

4. Procédé selon l'une ou plusieurs des revendications 1 à 3, dans lequel R₁ est un radical alkyle en C₄ à C₁₂.

5. Procédé selon l'une ou plusieurs des revendications 1 à 4, dans lequel R₁ est un groupe octyle ou isooctyle en position para.

6. Procédé selon l'une ou plusieurs des revendications 1 à 5, dans lequel R₂ est H ou CH₃.

7. Procédé selon l'une ou plusieurs des revendications 1 à 6, dans lequel n vaut 2.

8. Procédé selon l'une ou plusieurs des revendications 1 à 7, dans lequel R₃ est H et X est Cl.

9. Procédé selon l'une ou plusieurs des revendications 1 à 8, dans lequel les composés arylpoly(oxalkylés) de formule (2) et les benzyldiméthylamines de formule (3) sont utilisés selon un rapport en moles de 1:0,95 à 1:1,05.

10. Procédé selon l'une ou plusieurs des revendications 1 à 9, dans lequel la réaction de (2) et de (3) est mise en oeuvre à une température de 100 à 140°C, en particulier de 115 à 130°C, sous la pression spontanée du mélange réactionnel.

11. Procédé selon l'une ou plusieurs des revendications 1 à 10, **caractérisé en ce que** le solvant utilisé lors de la réaction de (2) et de (3) est séparé après la réaction, et le dérivé de l'arylpoly(oxalkyl)benzyldiméthylammonium de formule (1) est purifié par recristallisation.

12. Procédé selon la revendication 11, **caractérisé en ce que**, quand le solvant utilisé est l'eau lors de la réaction de (2) et de (3), l'eau, après la réaction, est séparée par exemple par une distillation azéotropique à l'aide de toluène ou de xylène, par séchage sous vide, par séchage par atomisation ou par séchage en lit fluidisé, le dérivé de l'arylpoly(oxalkyl)benzyldiméthylammonium de formule (1) étant purifié par recristallisation.
